# EUROPEAN PATENT APPLICATION

(11) **EP 4 501 401 A1**
(43) Date of publication of application: **05.02.2025**
(21) Application number: 24177444.7
(22) Date of filing: 22.05.2024
(51) Int. Cl.: A61N 7/00

(54) **IN VIVO LIQUID FLOW PROMOTION DEVICE**

(30) Priority: 31.07.2023 JP 2023124329
(71) Applicant: Ricoh Company, Ltd., Tokyo 143-8555 (JP)
(72) Inventor: HOSOYA, Toshihiko, Tokyo, 143-8555 (JP); MURAMATSU, Koichi, Tokyo, 143-8555 (JP); NONOYAMA, Yusuke, Tokyo, 143-8555 (JP)
(74) Representative: Marks & Clerk LLP

(57) **Abstract**

Provided is an in vivo liquid flow promotion device capable of safely and effectively promoting a liquid flow in a desired direction.

The in vivo liquid flow promotion device (1) includes an ultrasonic oscillator (2) that generates an ultrasonic wave, and an acoustic flow generation unit (3) that adjusts the phase of an ultrasonic wave generated by an ultrasonic oscillator (2) to focus the ultrasonic wave on a predetermined position, and that allows to generate an acoustic flow in in vivo fluid at a focused position by a pressure distribution generated in accordance with the phase.

## Description

### BACKGROUND

### Field of the Invention

The present invention relates to an in vivo liquid flow promotion device.

Priority is claimed on Japanese Patent Application No. 2023-124329, filed July 31, 2023, the content of which is incorporated herein by reference.

### Description of Related Art

It is known that the promotion of in vivo liquid flow of body fluids can treat certain diseases and enhance health. For example, by promoting blood circulation, improvement to stiff shoulders or low body temperature can be expected, and in a case of lymphatic fluid, fatigue may be reduced since liquid flow promotion assists in the removal of waste products. Furthermore, in a case of cerebrospinal fluid, it has recently been suggested that liquid flow promotion may assist in the treatment of diseases.

For example, there are a large number of people all over the world suffering from neurological diseases (depression, dementia, and the like). Antidepressants, for example, supply neurotransmitters as a general treatment method for depression, but there are also other causes of the onset of symptoms. Therefore, there is a need to establish new drugs that act on these causes or new treatment modalities that replace drugs.

As a new drug-free treatment method, promoting the liquid flow of cerebrospinal fluid, which is a liquid surrounding nerve cells in the brain, is considered. The cerebrospinal fluid is a liquid that surrounds nerve cells in the brain. In recent years, it has been clarified that the brain actively circulates cerebrospinal fluid during sleep to remove waste products. When this flow is weakened because of aging or the like, waste products accumulate around nerve cells and cause dementia by denaturing the cells themselves. In addition, it has been found that the deterioration of the flow of cerebrospinal fluid is also related to depression, and the improvement of the flow suggests the possibility of improving the symptoms. Furthermore, it has been found that the quality of sleep and brain aging are also related to the deterioration of the flow of cerebrospinal fluid. Therefore, in a case where the flow of cerebrospinal fluid can be controlled and promoted from the outside, there is a possibility to prevent or treat a neurological disease (depression, dementia) and to alleviate symptoms of brain aging.

Patent Document 1 discloses that the cerebrospinal fluid is locally expanded by the thermal energy generated by irradiating a predetermined site in the brain with a sound wave to induce convective flow and promote the liquid flow. However, the technique of Patent Document 1 needs to be carried out while suppressing the side effect due to heat generation, and there is a safety issue in that it is not possible to ensure effective liquid flow promotion. In addition, since the expansion occurs isotropically, a force may be applied in a direction that does not promote the liquid flow, thereby causing the cerebrospinal fluid to flow back.

### SUMMARY OF THE INVENTION

### [Problems to be solved by invention]

An object of the present invention is to provide an in vivo liquid flow promotion device capable of safely and effectively promoting a liquid flow in a desired direction.

### [Means for solving problems]

An aspect of the present invention discloses an in vivo liquid flow promotion device including an ultrasonic oscillator that generates an ultrasonic wave, and an acoustic flow generation unit that adjusts the phase of an ultrasonic wave generated by an ultrasonic oscillator to focus the ultrasonic wave on a predetermined position, and that allows generation of an acoustic flow in in vivo fluid at a focused position by a pressure distribution generated in accordance with the phase.

### [Effect of invention]

The present invention provides an in vivo liquid flow promotion device capable of safely and effectively promoting a liquid flow in a desired direction.

### BRIEF DESCRIPTION OF THE DRAWINGS

FIG. 1 is a schematic configuration view showing a first embodiment of an in vivo liquid flow promotion device according to the present invention.
FIG. 2 is a partial cross-sectional view of the in vivo liquid flow promotion device 1.
FIG. 3 is a schematic view showing a flow of cerebrospinal fluid in a brain.
FIG. 4 is a cross-sectional view of an acoustic refraction member 3G according to a second embodiment.
FIG. 5 is a perspective view of the acoustic refraction member 3G as viewed from the focused position S side.
FIG. 6 is a diagram showing a pressure distribution in the X direction of ultrasonic waves focused at the focused position S.
FIG. 7 is a diagram showing a pressure distribution in the Y direction of the ultrasonic waves focused at the focused position S.
FIG. 8 is a view showing a pressure distribution in an XY plane of the ultrasonic waves focused at the focused position S.
FIG. 9 is a view showing an experimental example in which ultrasonic waves are focused on a liquid.
FIG. 10 is a perspective view of an acoustic refraction member 3M according to a third embodiment.
FIG. 11 is a view showing a state in which the acoustic refraction member 3M generates a driving force in a space, by a vector.
FIG. 12 is an enlarged view of part of FIG. 11 as viewed from the side.
FIG. 13 is a schematic configuration view showing a fourth embodiment of an in vivo liquid flow promotion device according to the present invention.
FIG. 14 is a view showing a usage example of the in vivo liquid flow promotion device.
FIG. 15 is a view showing a usage example of the in vivo liquid flow promotion device.
FIG. 16 is a view showing a usage example of the in vivo liquid flow promotion device.
FIG. 17 is a view showing a usage example of the in vivo liquid flow promotion device.
FIG. 18 is a view showing a usage example of the in vivo liquid flow promotion device.
FIG. 19 is a view showing a usage example of the in vivo liquid flow promotion device.
FIG. 20 is a view showing a usage example of the in vivo liquid flow promotion device.

### DETAILED DESCRIPTION OF THE INVENTION

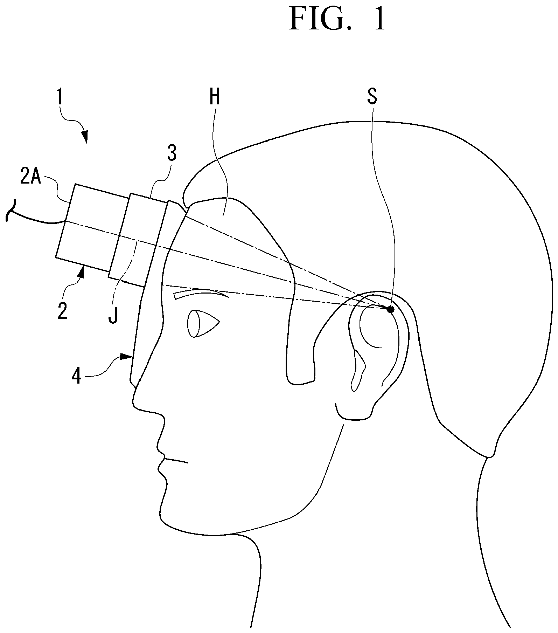

Hereinafter, embodiments of the in vivo liquid flow promotion device of the present invention will be described with reference to FIGs. 1 to 20.

The following embodiment is merely one aspect of the present invention, and is not to be considered as limiting the present invention thereto. Changes within the scope of the technical idea of the present invention are possible. In addition, in order to make each configuration easy to understand, the scale and number of each structure in the following drawings are different from those of the actual structure.

### [First embodiment of in vivo liquid flow promotion device]

FIG. 1 is a schematic configuration view showing a first embodiment of an in vivo liquid flow promotion device according to the present invention. The in vivo liquid flow promotion device 1 focuses and irradiates the ultrasonic wave at a predetermined position in the body and focused on the living body H, thereby generating an acoustic flow at the focused position S of the ultrasonic wave and promoting the liquid flow of the internal fluid in the direction of the flow of the fluid in vivo. The "flow of the fluid in vivo" means a vector in the advancing direction of the fluid in vivo. The "predetermined position" is a specific position in a path in which a fluid in vivo flows in vivo and can be appropriately set according to the desired effect. In FIG. 1, the in vivo liquid flow promotion device 1 is mounted on the patient's head, and the focused position S is set inside the patient's head (in the brain).

As shown in FIG. 1, the in vivo Liquid flow promotion device 1 includes an Ultrasonic oscillator 2, an acoustic flow generation unit 3, and a guide member 4.

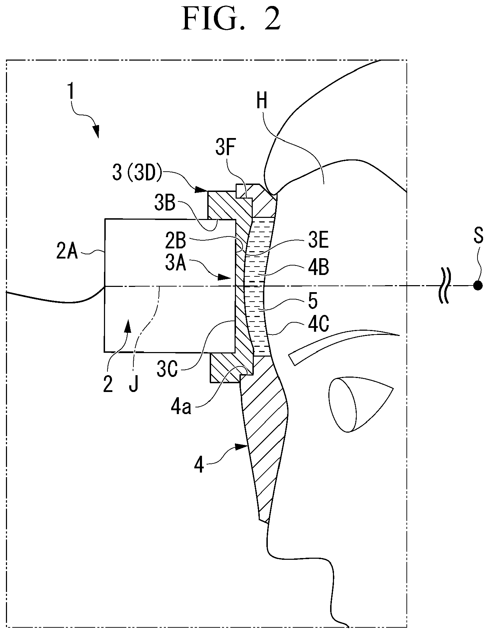

The ultrasonic oscillator 2 generates an ultrasonic wave in a direction in which the axis J extends. The ultrasonic oscillator 2 is housed inside a cylindrical cover 2A. FIG. 2 is a partial cross-sectional view of the in vivo liquid flow promotion device 1. As shown in FIG. 2, the ultrasonic oscillator 2 has a vibration surface 2B at the tip on the focused position S side.

The acoustic flow generation unit 3 adjusts the phase of the ultrasonic wave generated by the ultrasonic oscillator 2 and causes the ultrasonic waves to be focused on a predetermined position. The axis J is an imaginary line extending from the acoustic refraction member 3D (described later) of the acoustic refraction member 3 toward the focused position S. The ultrasonic waves emitted from the ultrasonic oscillator 2 advances in a direction along the axis J (a direction from the acoustic refraction member 3D toward the focused position S). Therefore, the direction may be referred to as a "advancing direction" in the following description.

The acoustic flow generation unit 3 has a columnar protrusion 3F on a side facing the focused position S. The acoustic flow generation unit 3 has a circular recess 3A on a side opposite to the focused position S. A tip of the cover 2A of the ultrasonic oscillator 2 on the side facing the focused position S is inserted into the recess 3A. The peripheral surface 3B of the recess 3Ais fitted to the outer periphery of the cover 2A. The bottom surface 3C of the recess 3A is in surface contact with the vibration surface 2B of the ultrasonic oscillator 2. The ultrasonic wave generated by the ultrasonic oscillator 2 is transmitted to the acoustic flow generation unit 3 through the vibration surface 2B and the bottom surface 3C.

The acoustic flow generation unit 3 of the present embodiment adjusts the phase of the ultrasonic wave by setting the direction that is uniform in the circumferential direction centered on the axis J and toward the focused position S in the radial direction centered on the axis J as the advancing direction. The acoustic flow generation unit 3 of the present embodiment has an acoustic refraction member 3D. The acoustic refraction member 3D has a concave surface 3E on a side facing the focused position S. The concave surface 3E has a curved surface shape for focusing the ultrasonic waves at the target position. In a case where the axis J is set at the center of the plan view of the acoustic refraction member 3D, the concave surface 3E is curved in a direction toward the focused position S in the advancing direction as going outward in a radial direction centered on the axis J extending in the advancing direction. The concave surface 3E is a spherical surface recessed from the surface of the acoustic refraction member 3D on the side facing the focused position S. The acoustic refraction member 3D refracts the ultrasonic wave incident from the ultrasonic oscillator 2 on the concave surface 3E, with a direction toward the focused position S as an advancing direction.

The material of the acoustic refraction member 3D is, as an example, a silicone rubber, and a PDMS (polydimethylsiloxane)-based silicone rubber is used. The acoustic impedance of PDMS is substantially the same as that of water, and thus it is possible to more suitably propagate ultrasonic waves.

The guide member 4 has a recess 4A, a housing portion 4B, and a joining portion 4C. The recess 4A has a circular shape and is located on the side opposite to the focused position S in the guide member 4. The protrusion 3F of the acoustic flow generation unit 3 is inserted into and fitted to the recess 4A. As the guide member 4, a silicone rubber or a resin member having a high hardness is used.

The housing portion 4B penetrates the guide member 4 along the axis J. The housing portion 4B is a circular through-hole centered on the axis J. The housing portion 4B is open toward the focused position S. The diameter of the housing portion 4B is larger than the diameter of the concave surface 3E. The concave surface 3E is formed to face the housing portion 4B. The housing portion 4B can house and hold the propagation liquid 5 through which the ultrasonic wave propagates. As the propagation liquid 5, water or a gel used in a general echo test is suitable.

The joining portion 4C is a region of the guide member 4 on the side facing the focused position S. The joining portion 4C surrounds the housing portion 4B. The joining portion 4C is joined to the living body H. The joining portion 4C of the present embodiment is joined, for example, from the forehead to the nose in the living body H. In a case where the joining portion 4C is joined to the living body H, the housing portion 4B is sealed. In a case where the joining portion 4C is joined to the living body H, the propagation liquid 5 is held in a state of being filled in the housing portion 4B. In a case where the joining portion 4C is joined to the living body H, the focused position S is defined as a target position. In other words, the guide member 4 is formed such that the focused position S is defined at the target position when the joining portion 4C is joined to the living body H.

A method for producing the guide member 4 is not particularly limited, but it is suitable to acquire facial shape data of a subject by 3D scanning and to produce the guide member 4 using the acquired facial shape data, for example, with a general-purpose 3D printer. By adopting this method, the subject can easily position the in vivo liquid flow promotion device 1 on the facial surface and use.

In order to promote the liquid flow in living body H using the in vivo liquid flow promotion device 1 having the above configuration, the joining portion 4C of the guide member 4 is joined to the facial surface of the subject who is the living body H. After that, the ultrasonic oscillator 2 generates ultrasonic waves. The generated ultrasonic waves are emitted from the forehead of the subject through the vibration surface 2B, the bottom surface 3C, the concave surface 3E, and the propagation liquid 5.

Here, in a case where the hair is present, the ultrasonic wave cannot be propagated into the brain, and thus it is necessary to shave the hair. For the subject, shaving the hair is accompanied by mental stress and is preferably avoided. In the present embodiment, since the hair does not need to be shaved by emitting the ultrasonic wave from the forehead, the embodiment is practically excellent. Regarding the irradiation position, for example, the ultrasonic wave may be emitted from a place where the hair is present as long as the liquid flow in the living body H can be promoted even if the hair is shaved.

The ultrasonic waves emitted from the forehead of the subject are focused at the focused position S in the advancing direction set by the acoustic refraction member 3D as shown in FIG. 1, and an acoustic flow is generated in the direction in which the axis J extends by the pressure distribution generated in accordance with the adjusted phase.

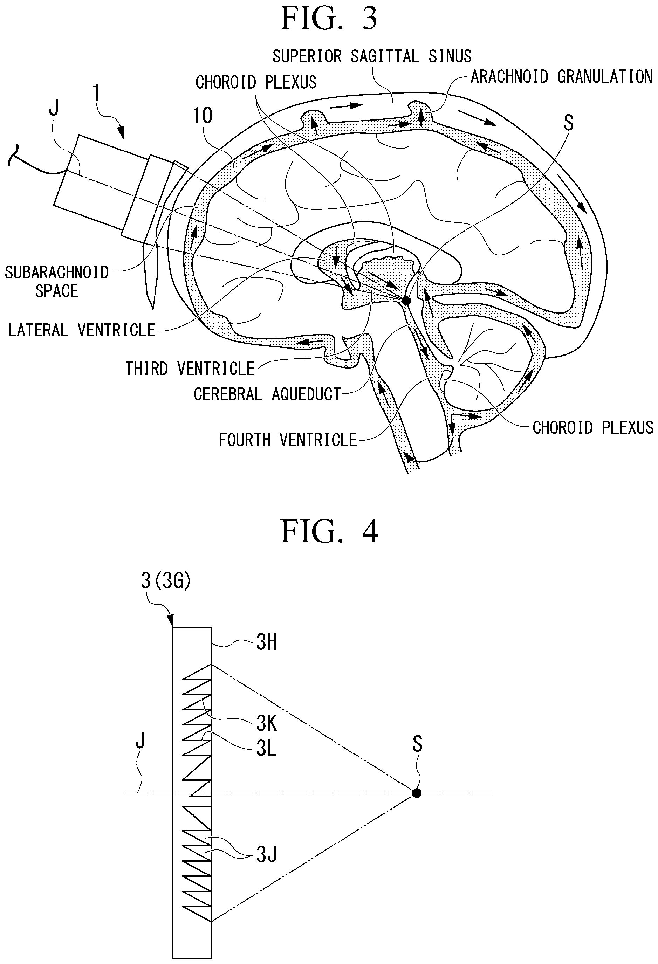

FIG. 3 is a schematic view showing a flow of cerebrospinal fluid in a brain.

As shown in FIG. 3, it is considered that the cerebrospinal fluid 10 oozes out from the choroid plexus, flows through the brain, is absorbed by the arachnoid granulation, and flows into the superior sagittal sinus, which is a blood vessel in the brain. In FIG. 3, the original flow direction of the cerebrospinal fluid 10 is indicated by an arrow. In addition, also in other views to be described below, in a schematic view similar to or corresponding to FIG. 3, the flow direction of the cerebrospinal fluid is indicated by an arrow.

The flow of the cerebrospinal fluid 10, which is in vivo fluid in the brain, oozes out from the choroid plexus of the lateral ventricle, advances to the third ventricle, passes through the cerebral aqueduct, and reaches the fourth ventricle. Thereafter, the cerebrospinal fluid 10 advances to the subarachnoid space, which is a gap between the brain and the arachnoid membrane that wraps the entire brain, and a part thereof advances to the occipital region, part thereof advances to the front side around the periphery of the spinal cord and to the frontal lobe.

The ultrasonic waves irradiated from the in vivo liquid flow promotion device 1 are irradiated to a target position (a predetermined position) of the focused position S near the entrance of the cerebral aqueduct. The acoustic flow generated at the focused position S by the irradiation with the ultrasonic wave is generated along the axis J within the third ventricle and toward the downstream cerebral aqueduct, and promotes the liquid flow in a form along the original flow. The irradiation direction (direction along the axis J) does not need to be completely parallel to the original flow direction of the cerebrospinal fluid 10. A vector component in the liquid flow direction may be included in the irradiation direction.

In this case, from the viewpoint of stably promoting the liquid flow, the irradiation direction of the ultrasonic wave (direction along the axis J) is preferably approximately -60° or more and +60° or less with respect to the flow direction of the cerebrospinal fluid 10. In addition, in a case where the irradiation power of the ultrasonic wave is about 10 w/cm² at the highest portion at the focused position S, the ultrasonic wave can be used in an empirically safe range.

In addition, the promotion of the liquid flow of the cerebrospinal fluid 10 can be confirmed by, for example, observing the liquid flow of the cerebrospinal fluid 10 at the focused position S (target position) using magnetic resonance imaging (MRI).

As described above, in the in vivo liquid flow promotion device 1 of the present embodiment, the ultrasonic oscillator 2 adjusts the phase of the ultrasonic wave generated to focus the ultrasonic waves and generates the acoustic flow in the fluid in vivo at the focused position S. Therefore, it is not necessary to perform the promotion while suppressing a side effect due to the heat generation, as in the case of promoting the liquid flow using the convective flow generated by the local expansion of the cerebrospinal fluid by the thermal energy.

Therefore, in the in vivo liquid flow promotion device 1 of the present embodiment, the liquid flow of the cerebrospinal fluid 10 in the brain can be safely and effectively promoted, and the effect of promoting the removal of waste products can be obtained.

In addition, in the in vivo liquid flow promotion device 1 of the present embodiment, since the liquid flow of the fluid in vivo is promoted by the acoustic flow, the liquid flow can be promoted in a desired direction without causing a backflow as in a case where the cerebrospinal fluid is locally expanded.

### [Second embodiment of in vivo liquid flow promotion device]

Subsequently, a second embodiment of the in vivo liquid flow promotion device 1 will be described with reference to FIGs. 4 to 9. In these drawings, the same reference numerals are given to the same elements as constituent elements of the first embodiment shown in FIGs. 1 to 3, and the description thereof will be omitted.

In the in vivo liquid flow promotion device 1 of the second embodiment, the configuration of the acoustic refraction member is different from that of the first embodiment.

The in vivo liquid flow promotion device 1 has an acoustic refraction member 3G as the acoustic flow generation unit 3.

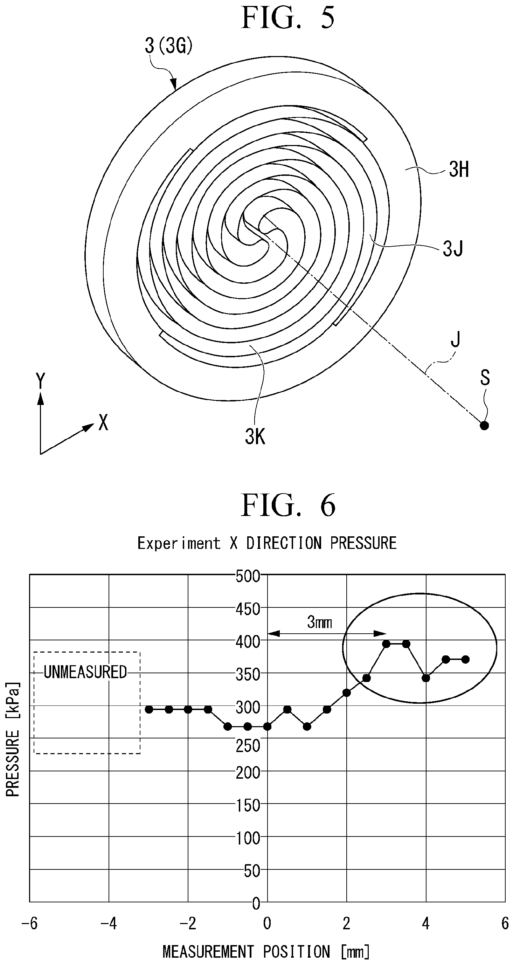

FIG. 4 is a cross-sectional view of the acoustic refraction member 3G. FIG. 5 is a perspective view of the acoustic refraction member 3G as viewed from the focused position S side.

As shown in FIG. 4, the acoustic refraction member 3G has a groove 3J recessed from the surface 3H facing the focused position S. The cross section of the groove 3J along the radial direction has a V-shape. The V-shaped groove 3J is formed by the inclined surface 3K and the vertical surface 3L. In a case where the axis J is set to be centered on the plan view of the acoustic refraction member 3G, the inclined surface 3K is inclined in a direction inward in the radial direction centered on the axis J as going away from the surface 3H. The vertical surface 3L extends in a direction orthogonal to the surface 3H and away from the surface 3H. The inclined surface 3K and the vertical surface 3L, which form the adjacent grooves 3J in the radial direction, intersect with each other at the position of the surface 3H in the direction along the axis J.

In a case of being viewed from the side of the focused position S, the inclined surface 3K is arranged in a spiral shape extending in a direction toward one side in the circumferential direction centered on the axis J as going outward in the radial direction from the axis J. The inclined surface 3K is arranged in a spiral shape extending in the clockwise direction as going outward in the radial direction. The inclined surface 3K may be configured to be arranged in a spiral shape extending in the counterclockwise direction as going outward in the radial direction.

A plurality of inclined surfaces 3K are arranged at predetermined intervals in the circumferential direction. Four inclined surfaces 3K are arranged at intervals of 90° in the circumferential direction.

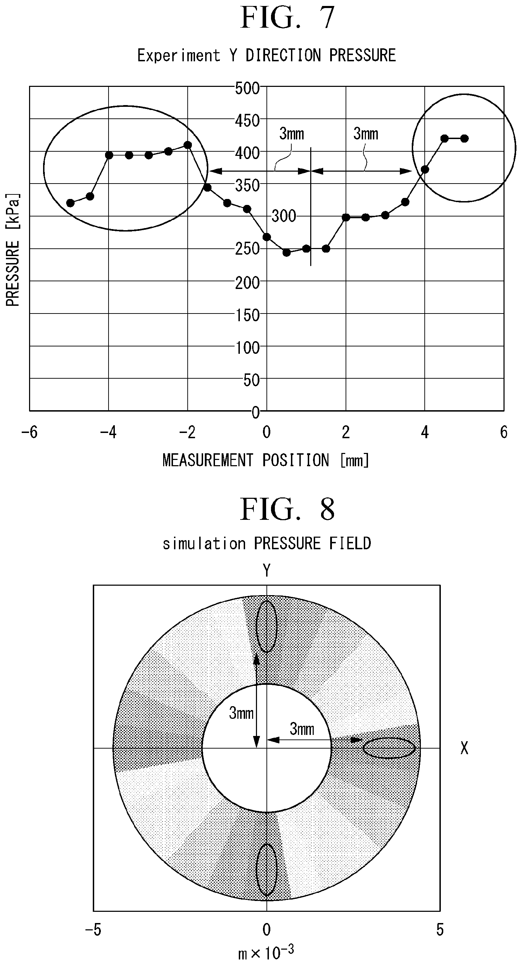

FIG. 6 is a diagram showing an experimental result of a pressure distribution (pressure field) in the X direction of the ultrasonic waves focused at the focused position S in a case where directions parallel to the surface 3H and orthogonal to each other are defined as the X direction and the Y direction in the acoustic refraction member 3G. FIG. 7 is a diagram showing an experimental result of a pressure distribution (pressure field) in the Y direction of the ultrasonic waves focused at the focused position S. FIG. 8 is a diagram showing a numerical simulation result of a pressure distribution (pressure field) in an XY plane of the ultrasonic waves focused at the focused position S.

As shown in FIG. 6, it was confirmed that at the focused position S, there is a place where the pressure increases at a position of a radius of 3 mm on the +X side from the axis J, and the measurement is performed in the X direction. It can be presumed that there is a place where the pressure increases similarly at a position of a radius of 3 mm on the -X side from the axis J, and the measurement is not performed. Similarly, as shown in FIG. 7, it was confirmed that there are places where the pressure increases at positions of a radius of 3 mm from the axis J on both sides in the Y direction. From the experimental results, it was presumed that a ring-shaped pressure field as shown in FIG. 8 was formed.

In addition, as shown in FIG. 8, at the focused position S of the ultrasonic wave, the pressure of the pressure field on the X-axis and the Y-axis is the largest, and a circumferential pressure distribution in which the pressure of the pressure field decreases as going in the clockwise direction is generated.

Therefore, the pressure distribution is generated in the circumferential direction centered on the axis J at the focused position S of the ultrasonic waves, and thus the acoustic flow in the circumferential direction can be generated.

As a result, the cerebrospinal fluid 10 located at the focused position S can be caused to generate a vortex flow by a force acting in a rotating direction in accordance with the pressure distribution in the circumferential direction centered on the axis J.

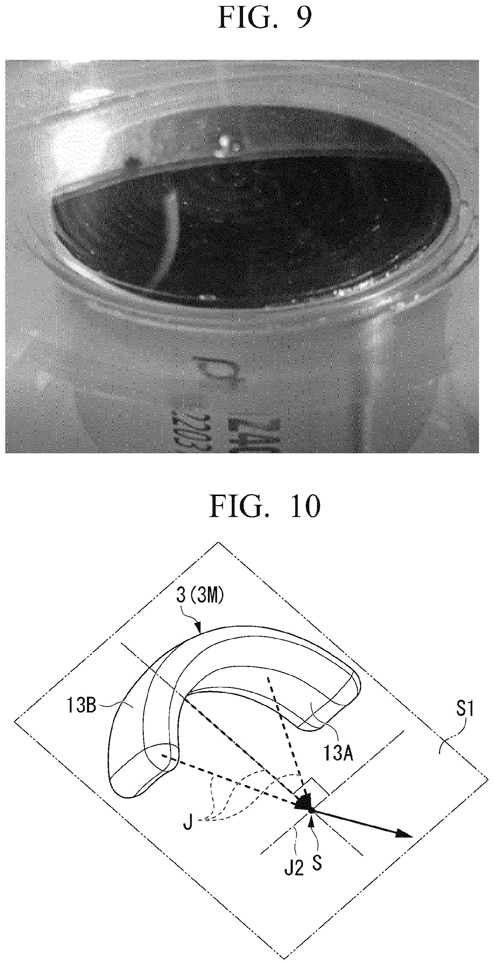

FIG. 9 is a view showing an experimental example in which ultrasonic waves are focused on a liquid using the acoustic refraction member 3G. As indicated by the arrow in FIG. 9, it was possible to observe that the liquid rotated in the clockwise direction.

In the in vivo liquid flow promotion device 1 of the present embodiment, in addition to obtaining the same operation and effect as those of the first embodiment, the acoustic flow in the rotating direction can be generated in the fluid in vivo, and the liquid flow can be further promoted.

### [Third embodiment of in vivo liquid flow promotion device]

Subsequently, a third embodiment of the in vivo liquid flow promotion device 1 will be described with reference to FIGs. 10 to 12. In these drawings, the same reference numerals are given to the same elements as constituent elements of the first embodiment shown in FIGs. 1 to 8, and the description thereof will be omitted or simplified.

In the in vivo liquid flow promotion device 1 of the third embodiment, the configuration of the acoustic refraction member is different from that of the first embodiment.

The in vivo liquid flow promotion device 1 has an acoustic refraction member 3M as the acoustic flow generation unit 3.

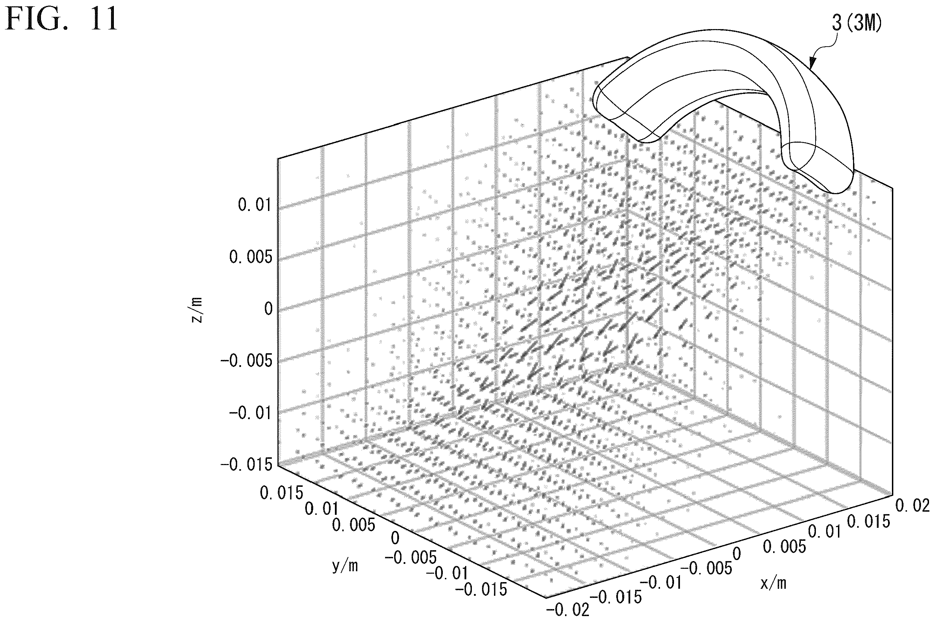

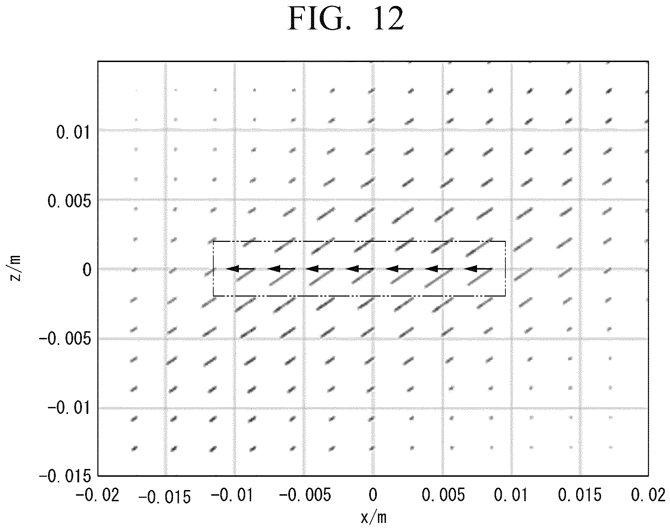

FIG. 10 is a perspective view of the acoustic refraction member 3M. FIG. 11 is a view showing a state in which the acoustic refraction member 3M generates a driving force in a space, by a vector. FIG. 12 is an enlarged view of a part of FIG. 11 as viewed from the side.

As shown in FIG. 10, the acoustic refraction member 3M in the present embodiment has an inner peripheral surface 13A and an outer peripheral surface 13B. The inner peripheral surface 13A and the outer peripheral surface 13B are peripheral surfaces centered on a second axis J2 orthogonal to a virtual plane S1 including the axis J in a plan view. The second axis J2 is in a relationship orthogonal to each of the three axes J shown in FIG. 10. The acoustic refraction member 3M is formed in an arc shape where the inner peripheral surface 13A faces the focused position S.

The ultrasonic waves advancing through the acoustic refraction member 3M having the above configuration are focused at the focused position S in a case of being viewed from the second axis J2. On the other hand, in a case of being viewed from the lateral direction orthogonal to the second axis J2, the ultrasonic wave emitted from the inner peripheral surface 13A is not focused because the ultrasonic wave travels orthogonal to the generating line parallel to the second axis J2.

Therefore, as shown in FIG. 11, in the ultrasonic waves emitted from the acoustic refraction member 3M, a driving force vector directed from the upper right to the lower left, but as shown in FIG. 12, the driving force vector is in a state of being parallel to the direction along the second axis J2. In this case, the component of the driving force vector directed to the left in the figure is generated in a linear shape as indicated by the arrow surrounded by the quadrangle of the two-dot chain line.

In the in vivo liquid flow promotion device 1 of the present embodiment, the same operation and effect as those of the first embodiment can be obtained, and effective liquid flow promotion can be achieved by irradiating a site where it is desired to generate a linear flow.

### [Fourth embodiment of in vivo liquid flow promotion device]

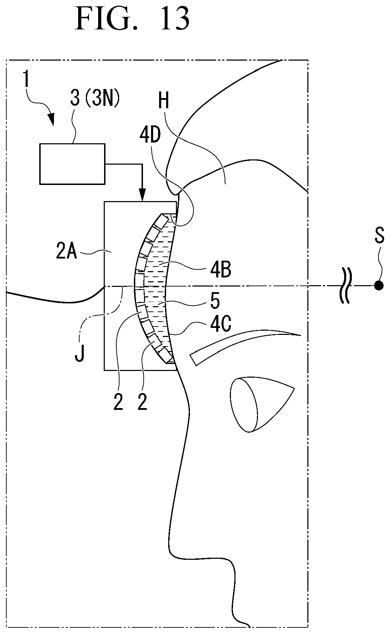

Subsequently, a fourth embodiment of the in vivo liquid flow promotion device 1 will be described with reference to FIG. 13. In the drawing, the same reference numerals are given to the same elements as constituent elements of the first embodiment shown in FIGs. 1 to 3, and the description thereof will be omitted or simplified.

In the in vivo liquid flow promotion device 1 of the third embodiment, the configurations of the ultrasonic oscillator 2, the acoustic flow generation unit 3, and the guide member 4 are different from these of the first embodiment.

FIG. 13 is a schematic configuration view showing a fourth embodiment of the in vivo liquid flow promotion device 1.

As shown in FIG. 13, the guide member 4 in the present embodiment is provided at the tip of the cover 2A on the focused position S side. The guide member 4 has a housing portion 4B, a joining portion 4C, and a cup portion 4D. The cup portion 4D has a cup shape that increases in diameter from the tip of the cover 2A toward the living body H. The joining portion 4C is provided at the tip of the cup portion 4D. In addition, the housing portion 4B is provided inside the cup portion 4D.

The ultrasonic oscillator 2 has a size of, for example, about several mm. A plurality of the ultrasonic oscillators 2 are arranged at positions facing the housing portion 4B. Each of the plurality of ultrasonic oscillators 2 can independently generate ultrasonic waves. The ultrasonic oscillator 2 is arranged in an array at the tip of the cover 2A facing the housing portion 4B. The ultrasonic oscillator 2 is arranged in an array on an inner peripheral surface of the cup portion 4D facing the housing portion 4B.

The acoustic flow generation unit 3 has a control unit 3N. The control unit 3N individually controls the phases of the ultrasonic waves generated by the plurality of ultrasonic oscillators 2. The control unit 3N individually controls the phases of the ultrasonic waves generated by the plurality of ultrasonic oscillators 2, and thus the acoustic flow can be generated by the pressure distribution generated in accordance with the phase.

For example, the control unit 3N individually controls the phase of the ultrasonic wave generated by the ultrasonic oscillator 2, and a pressure distribution same to the pressure distribution generated by the concave surface 3E of the acoustic refraction member 3D shown in FIG. 2 is generated, whereby the ultrasonic waves can be focused on the focused position S in a point shape.

In addition, the control unit 3N individually controls the phase of the ultrasonic wave generated by the ultrasonic oscillator 2, and a pressure distribution same to or similar to the pressure distribution shown in FIG. 8 generated by the inclined surface 3K of the acoustic refraction member 3G shown in FIGs. 4 and 5 is generated, whereby the acoustic flow is generated in which a vortex flow is generated by a force acting in the rotating direction in accordance with the pressure distribution in the circumferential direction.

Furthermore, the control unit 3N individually controls the phase of the ultrasonic wave generated by the ultrasonic oscillator 2, and a pressure distribution same to or similar to the pressure distribution generating the driving force vector shown in FIGs. 11 and 12 is generated, whereby the acoustic flow causing to generate a linear flow can be generated.

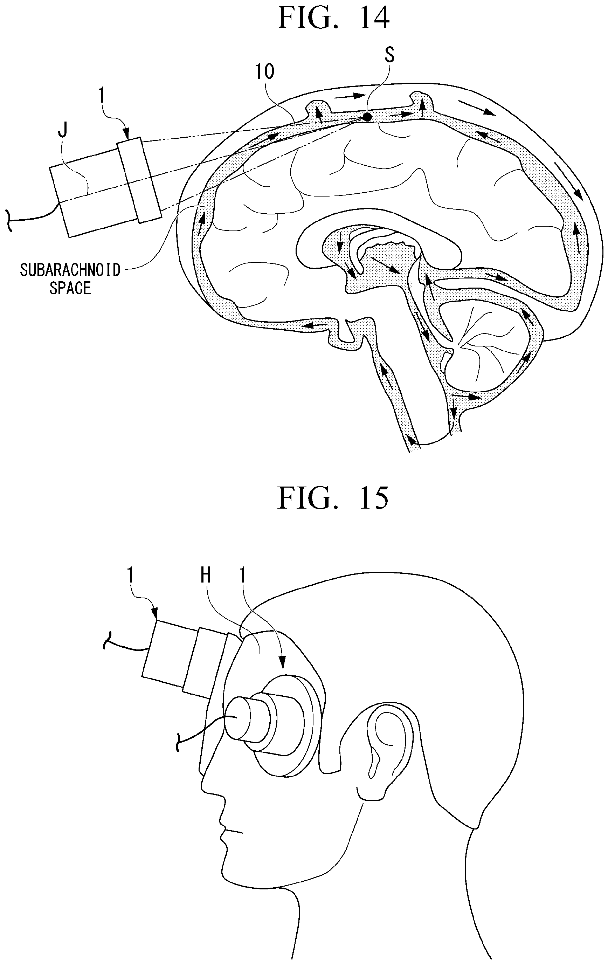

[Usage example of in vivo liquid flow promotion device 1] FIG. 14 is a view showing a usage example of the in vivo liquid flow promotion device 1.

In the first embodiment, as shown in FIG. 3, the vicinity of the entrance of the cerebral aqueduct was irradiated with the ultrasonic wave as the target position of the focused position S. As shown in FIG. 14, the flow from the front side to the rear side of the head can be promoted by irradiating the subarachnoid space from the forehead with ultrasonic wave at the focused position S.

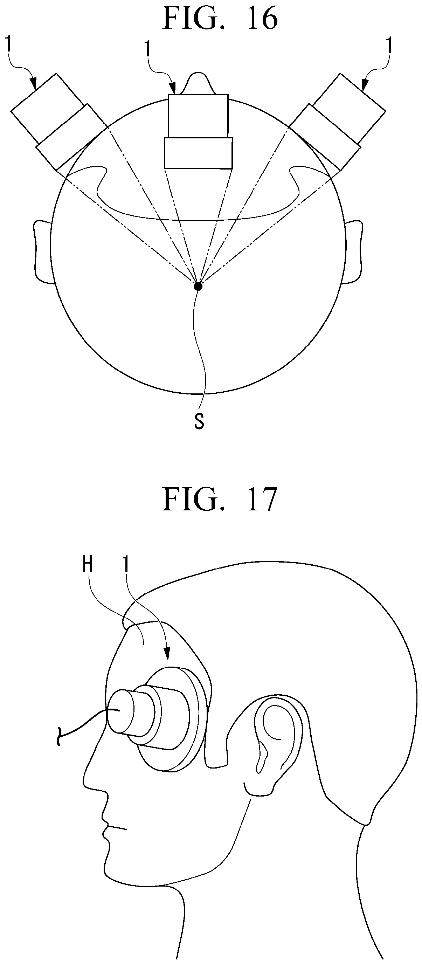

FIGs. 15 and 16 are views showing a usage example of another in vivo liquid flow promotion device 1.

In the first embodiment, as shown in FIG. 3, the ultrasonic wave was emitted from the forehead using the in vivo liquid flow promotion device 1. As shown in FIGs. 15 and 16, a configuration may be adopted in which the ultrasonic waves are emitted from the left and right temple portions in addition to the forehead using a plurality of the in vivo liquid flow promotion devices 1.

By adopting this configuration, the effect of the liquid flow can be further enhanced. In this case, the focused positions S may be the same, or the focused positions S at different positions may be irradiated with the ultrasonic waves by the plurality of the in vivo liquid flow promotion devices 1.

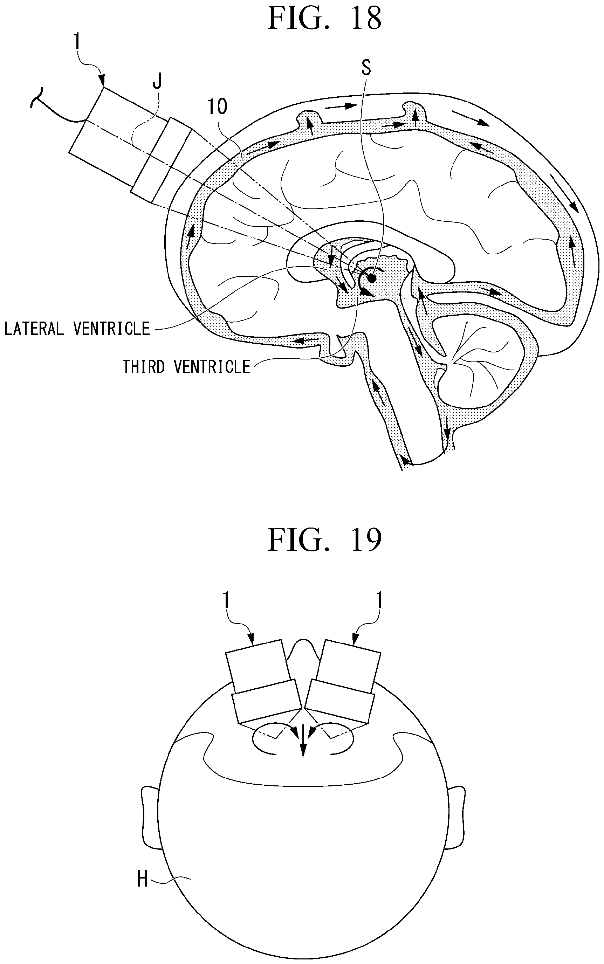

FIGs. 17 and 18 are views showing a usage example of another in vivo liquid flow promotion device 1.

In the second embodiment, a configuration is shown as an example, in which the vicinity of the entrance of the cerebral aqueduct was irradiated with the ultrasonic wave as the target position of the focused position S and a vortex flow is generated by a force acted in a rotating direction to the cerebrospinal fluid 10. As shown in FIG. 17, the ultrasonic wave may be emitted from the vicinity of the temple portion by the in vivo liquid flow promotion device 1 described in the second embodiment, or as shown in FIG. 18, the third ventricle may be irradiated with ultrasonic wave as the target position of the focused position S.

By adopting this configuration, the flow of the cerebrospinal fluid 10 rotating in the third ventricle can be generated. As a result, the inflow of the cerebrospinal fluid 10 from the lateral ventricle can be promoted.

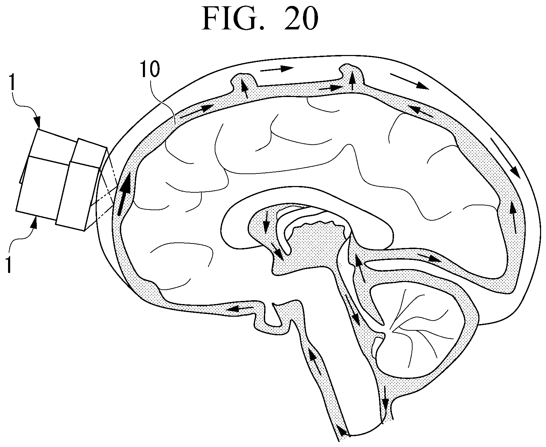

FIGs. 19 and 20 are views showing a usage example of another in vivo liquid flow promotion device 1.

In the second embodiment, a configuration is shown as an example, in which one in vivo liquid flow promotion device 1 is used and a vortex flow is generated by a force acted in a rotating direction to the cerebrospinal fluid 10. As shown in FIG. 19, two in vivo liquid flow promotion devices 1 are arranged on the left and right sides with the center of the forehead interposed therebetween. By the two in vivo liquid flow promotion devices 1, a force is acted on the cerebrospinal fluid 10 in opposite to a rotating direction to generate a vortex flow.

By adopting this configuration, it is possible to generate a flow of the cerebrospinal fluid 10 that travels straight in a central portion between the two in vivo liquid flow promotion devices 1. With this configuration, as shown in FIG. 20, it is possible to promote the liquid flow of the cerebrospinal fluid 10 flowing in the shallow portion of the forehead portion.

In addition, the usage example of the in vivo liquid flow promotion device 1 described with reference to FIGs. 14 to 20 can also be used by using the in vivo liquid flow promotion device 1 described as the fourth embodiment with reference to FIG. 13.

That is, the control unit 3N controls the phases of the ultrasonic waves generated by the plurality of ultrasonic oscillators 2, and the pressure distribution same as or similar to the pressure distribution generated in accordance with the phase adjusted by the acoustic flow generation unit 3 in each usage example of the in vivo liquid flow promotion device 1 described with reference to FIGs. 14 to 20, whereby the same usage result can be obtained.

Although preferred embodiments of the present invention have been described above with reference to the accompanying drawings, it goes without saying that the present invention is not limited to the examples. The variety of shapes, combinations, and the like of the individual constituent members described in the above-described examples are simply examples, and a variety of modifications are permitted based on design requirements and the like without departing from the gist of the present invention.

In the above embodiment, a configuration in which the fluid in vivo is the cerebrospinal fluid 10 is shown as an example, but the present invention is not limited to this configuration. Examples of the fluid in vivo include blood, lymphatic fluid, and the like, in addition to the cerebrospinal fluid 10. In particular, in the blood, an effect of promoting blood circulation is exhibited, and in the lymphatic fluid and the cerebrospinal fluid, an effect of removing waste products is exhibited.

In the above embodiment, a configuration is shown as an example, in which by the in vivo liquid flow promotion device 1, the head portion of the living body H is irradiated with the ultrasonic wave, but the present invention is not limited to this configuration. As the irradiation position in the living body H in a case of obtaining the effect of liquid flow promotion of the cerebrospinal fluid 10, a configuration may be adapted, in which for example, the spinal portion in addition to the head portion is irradiated.

In addition, as the position of the living body H irradiated with the ultrasonic wave by in vivo liquid flow promotion device 1, at least one or more of the facial surface, the surrounding parts of the neck, and the back is preferable.

By adopting this configuration, since shaving the hair is not required in a case of obtaining the effect of liquid flow promotion of the cerebrospinal fluid 10, it is possible to reduce the psychological stress of the subject.

### The present invention includes the following aspects.

[1] An in vivo liquid flow promotion device including an ultrasonic oscillator that generates an ultrasonic wave, and an acoustic flow generation unit that adjusts a phase of the ultrasonic wave generated by the ultrasonic oscillator to focus the ultrasonic wave on a predetermined position, and that allows to generate an acoustic flow in in vivo fluid at a focused position by a pressure distribution generated in accordance with the phase.
[2] The in vivo liquid flow promotion device according to [1], in which the acoustic flow generation unit has an acoustic refraction member.
[3] The in vivo liquid flow promotion device according to [2], in which the acoustic refraction member has a concave surface that is curved in a direction toward the focused position as going outward in a radial direction centered on an axis extending from the acoustic refraction member toward the focused position.
[4] The in vivo liquid flow promotion device according to [2], in which the acoustic refraction member has an inclined surface that is inclined in a direction inward in a radial direction centered on an axis extending from the acoustic refraction member toward the focused position as going away from a surface facing the focused position, and the inclined surface is arranged in a spiral shape extending in a direction toward one side in a circumferential direction centered on the axis as going outward in the radial direction from the axis.
[5] The in vivo liquid flow promotion device according to [2], in which the acoustic refraction member has an inner peripheral surface and an outer peripheral surface, which are centered on a second axis that is located on a side of the focused position and is orthogonal to a virtual plane including an axis extending from the acoustic refraction member to the focused position, and the inner peripheral surface is formed in an arc shape facing the focused position.
[6] The in vivo liquid flow promotion device according to [1], in which a plurality of the ultrasonic oscillators are arranged in an array, and the acoustic flow generation unit has a control unit that individually controls the phases of the ultrasonic waves generated by the plurality of ultrasonic oscillators.
[7] The in vivo liquid flow promotion device according to any one of [1] to [6], further including a guide member having a housing portion that is open toward the focused position and houses a propagation liquid through which the ultrasonic wave propagates, and a joining portion that surrounds the housing portion and defines the focused position as a target position in a case of being joined to a living body.

### EXPLANATION OF REFERENCES

1: in vivo liquid flow promotion device
2: ultrasonic oscillator
3: acoustic flow generation unit
3D, 3G, 3M: acoustic refraction member
3E: concave surface
3K: inclined surface
4: guide member
4B: housing portion
4C: joining portion
5: propagation liquid
13A: inner peripheral surface
13B: outer peripheral surface
H: living body
J: axis
J2: second axis
S: focused position

[Prior art documents] [Patent Document] [0067] [Patent Document 1] Japanese Patent No. 6585836

## Claims

1. An in vivo liquid flow promotion device comprising:
an ultrasonic oscillator that generates an ultrasonic wave; and
an acoustic flow generation unit that adjusts the phase of an ultrasonic wave generated by an ultrasonic oscillator to focus the ultrasonic wave on a predetermined position, and that allows generation of an acoustic flow in in vivo fluid at a focused position by a pressure distribution generated in accordance with the phase.

2. The in vivo liquid flow promotion device according to claim 1,
wherein the acoustic flow generation unit has an acoustic refraction member.

3. The in vivo liquid flow promotion device according to claim 2,
wherein the acoustic refraction member has a concave surface that is curved in a direction toward the focused position as going outward in a radial direction centered on an axis extending from the acoustic refraction member toward the focused position.

4. The in vivo liquid flow promotion device according to claim 2 or 3,
wherein the acoustic refraction member has an inclined surface that is inclined in a direction inward in a radial direction centered on an axis extending from the acoustic refraction member toward the focused position as going away from a surface facing the focused position, and
the inclined surface is arranged in a spiral shape extending in a direction toward one side in a circumferential direction centered on the axis as going outward in the radial direction from the axis.

5. The in vivo liquid flow promotion device according to claim 2, 3 or 4,
wherein the acoustic refraction member has an inner peripheral surface and an outer peripheral surface, which are centered on a second axis that is located on a side of the focused position and is orthogonal to a virtual plane including an axis extending from the acoustic refraction member to the focused position, and
the inner peripheral surface is formed in an arc shape facing the focused position.

6. The in vivo liquid flow promotion device according to any one of claims 1 to 5,
wherein a plurality of the ultrasonic oscillators are arranged in an array, and
the acoustic flow generation unit has a control unit that individually controls the phases of the ultrasonic waves generated by the plurality of ultrasonic oscillators.

7. The in vivo liquid flow promotion device according to any one of claims 1 to 6, further comprising:
a guide member having a housing portion that is open toward the focused position and houses a propagation liquid through which the ultrasonic wave propagates, and a joining portion that surrounds the housing portion and defines the focused position as a target position in a case of being joined to a living body.
